# EUROPÄISCHE PATENTANMELDUNG

(11) **EP 1 917 909 A1**
(43) Veröffentlichungstag der Anmeldung: **07.05.2008**
(21) Anmeldenummer: 06122214.7
(22) Anmeldetag: 12.10.2006
(51) Int. Cl.: A61B 5/15

(54) **Probengewinnungssystem und Verfahren zum Gewinnen einer flüssigen Probe**

(71) Anmelder: Roche Diagnostics GmbH, 68305 Mannheim (DE); F. Hoffmann-la Roche AG, 4070 Basle (CH)
(72) Erfinder: Die Erfindernennung liegt noch nicht vor
(74) Vertreter: Dick, Alexander

(57) **Zusammenfassung**

Die Erfindung bezieht sich auf ein Probcngcwinnungssystem und ein Vcrfahrcn zum Gewinnen einer flüssigen Probe. Das erfindungsgemäße Probengewinnungssystem enthält mindestens ein analytisches Hilfsmittel (1), wobei das analytische Hilfsmittel (1) eine Lanzette (3) und ein Testelement (4) enthält und das Testelement (4) ein Testfeld (5) zur Analyse der flüssigen Probe aufweist. Das Probengcwinnungssystem enthält ein Kopplungselement (7), das nacheinander in einer ersten Position des analytischen Hilfsmittels (1) an die Lanzette (3) und in einer zweiten Position des analytischen Hilfsmittels (1) an das Testelement (4) koppelbar ist. Das Probengewinnungssystem enthält ferner eine Antriebseinheit (8), um eine Bewegung des Kopplungselements (7) aus einer Ruheposition (22) in eine ausgelenkte Position (23) anzutreiben, so dass die Bewegung des Kopplungselements (7) auf die daran angekoppelte Lanzette (3) zum Ausführen einer Stechbewegung oder auf das daran angekoppelte Testelement (4) zum Ausführen einer Probenaufnahmebewegung übertragen wird.

## Beschreibung

Die Erfindung bezieht sich auf ein Probengewinnungssystem und ein Verfahren zum Gewinnen einer flüssigen Probe, insbesondere einer Körperflüssigkeit wie Blut oder interstitielle Flüssigkeit. Die Probengewinnung umfasst dabei die zwei Schritte des Erzeugens einer Perforation der Haut durch Einstechen einer Lanzette, so dass aus der Perforation die Probenflüssigkeit austritt, und das Aufnehmen der Probe auf ein Testelement, auf dem sie in einem Testfeld analysiert werden kann.

Die Untersuchung von Blutproben oder von interstitieller Flüssigkeit ermöglichen in der klinischen Diagnostik das frühzeitige und zuverlässige Erkennen von pathologischen Zuständen sowie die gezielte und fundierte Kontrolle von Körperzuständen. Die medizinische Diagnostik setzt stets die Gewinnung einer Probe aus Blut oder interstitieller Flüssigkeit des zu untersuchenden Individuums voraus.

Zur Gewinnung der Probe kann die Haut z. B. an der Fingerbeere oder dem Ohrläppchen der zu untersuchenden Person mit Hilfe einer sterilen, scharfen Lanzette perforiert werden, um so einige wenige Mikroliter Blut oder weniger für die Analyse zu gewinnen. Insbesondere eignet sich diese Methode für die Analyse einer Probe, die unmittelbar nach der Probengewinnung durchgeführt wird.

Vor allem im Bereich des so genannten "Home-Monitoring", also dort, wo medizinische Laien selbst einfache Analysen des Bluts oder der interstitiellen Flüssigkeit durchführen, und dort insbesondere für die regelmäßige, mehrmals täglich durchzuführende Blutgewinnung durch Diabetiker für die Kontrolle der Blutglukosekonzentration, werden Lanzetten und dazu passende Geräte (so genannte Stechhilfen) angeboten, die eine möglichst schmerzarme und reproduzierbare Probengewinnung ermöglichen. Solche Lanzetten und Geräte (Stechhilfen) sind z. B. Gegenstand von WO-A 98/48695, US 4,442,836, US 5,554,166 oder WO 2006/013045 A1.

Die eigenhändige Blutzuckerbestimmung ist heute eine weltweit verbreitete Methode in der Diabetes-Kontrolle. Blutzuckergeräte im Stand der Technik bestehen aus einem Analysegerät, in das ein Testelement (Teststreifen) eingeführt wird. Das Testelement wird mit einem Tropfen einer Probe in Kontakt gebracht, der zuvor mittels einer Stechhilfe z. B. aus der Fingerbeere gewonnen wurde.

Zur Analyse von Flüssigproben, beispielsweise Körperflüssigkeiten wie Blut oder Urin, werden häufig Analysegeräte verwendet, bei denen sich die zu analysierende Probe auf einem Testfeld eines Testelements befindet und in dem Testfeld gegebenenfalls mit einer oder mehreren Reagenzien reagiert, bevor sie analysiert wird. Die optische, insbesondere photometrische, und die elektrochemische Auswertung von Testelementen stellen die gebräuchlichsten Verfahren zur schnellen Bestimmung der Konzentration von Analyten in Proben dar. Analysesysteme mit Testelementen zur Probenanalyse werden allgemein im Bereich der Analytik, der Umweltanalytik und vor allem im Bereich der medizinischen Diagnostik eingesetzt. Insbesondere im Bereich der Blutglukosediagnostik aus Kapillarblut besitzen Testelemente, die photometrisch oder elektrochemisch ausgewertet werden, einen großen Stellenwert.

Es gibt verschiedene Formen von Testelementen. Bekannt sind zum Beispiel im Wesentlichen quadratische Plättchen, die auch als Slides bezeichnet werden, in deren Mitte sich ein mehrschichtiges Testfeld befindet. Diagnostische Testelemente, die streifenförmig ausgebildet sind, werden als Teststreifen bezeichnet. Im Stand der Technik sind Testelemente umfassend beschrieben, beispielsweise in den Dokumenten CA 2311496 A1, US 5,846,837 A, US 6,036,919 A oder WO 97/02487.

Weitere im Stand der Technik bekannte mehrschichtige Testelemente sind Analysebänder mit einer Vielzahl von Testfeldern, die in einer Kassette aufgewickelt zur Verwendung in einem Analysegerät bereitgestellt werden. Solche Kassetten und Analysebänder werden zum Beispiel in den Dokumenten DE 103 32 488 A1, DE 103 43 896 A1, EP 1 424 040 A1, WO 2004/056269 A1 und US 2006/0002816 A1 beschrieben.

Die zahlreichen Systemkomponenten (Lanzette, Stechhilfe, Testelement und Analysegerät) benötigen viel Platz und bedingen eine relativ komplexe Handhabung. Inzwischen gibt es auch Systeme mit einem höheren Grad der Integration und damit einer einfacheren Handhabung, bei denen z. B. die Testelemente im Analysegerät magaziniert und für die Messung zur Verfügung gestellt werden. Ein nächster Schritt der Miniaturisierung ist beispielsweise durch die Integration von mehreren Funktionen beziehungsweise Funktionselementen in einem einzigen analytischen Hilfsmittel (Disposable) zu erreichen. Durch geeignete Kombination von Stechvorgang und sensorischer Analytkonzentrations-Erfassung auf einem Testelement lässt sich beispielsweise der Bedienablauf deutlich vereinfachen.

Aus WO 2005/107596 A2 ist das Bereitstellen einer Vielzahl voneinander beabstandeter Lanzetten auf einem Band bekannt. Gemäß einer Ausführungsvariante trägt das Band nicht nur die Lanzetten, sondern auch eine Vielzahl von Testelementen, die jeweils einer der Lanzetten zugeordnet sind. Dabei handelt es sich daher um ein Band mit einer Vielzahl von zueinander beabstandet angeordneten analytischen Hilfsmitteln, die eine Integration von Stechvorgängen und Probenaufnahmevorgängen in einem Probengewinnungssystem ermöglichen.

Aufgabe der vorliegenden Erfindung ist es, ein Probengewinnungssystem und ein Verfahren zum Gewinnen einer flüssigen Probe bereitzustellen, die die Funktionen des Einstechens der Lanzette eines analytischen Hilfsmittels und des Aufnehmens der flüssigen Probe auf einem Testelement des analytischen Hilfsmittels integrieren. Insbesondere ist es Aufgabe der vorliegenden Erfindung, eine hohe Integration und eine möglichst geringe Baugröße eines Probengewinnungssystems zu erreichen.

Diese Aufgabe wird erfindungsgemäß gelöst durch ein Probengewinnungssystem zum Gewinnen einer flüssigen Probe, enthaltend mindestens ein analytisches Hilfsmittel, wobei das analytische Hilfsmittel eine Lanzette und ein Testelement enthält und das Testelement ein Testfeld zur Analyse der flüssigen Probe aufweist. Das Probengewinnungssystem enthält ein Kopplungselement, das nacheinander in einer ersten Position des analytischen Hilfsmittels an die Lanzette und in einer zweiten Position des analytischen Hilfsmittels an das Testelement koppelbar ist. Ferner enthält das Probengewinnungssystem eine Antriebseinheit, um eine Bewegung des Kopplungselements aus einer Ruheposition in eine ausgelenkte Position anzutreiben, so dass die Bewegung des Kopplungselements auf die daran angekoppelte Lanzette zum Ausführen einer Probenaufnahmebewegung oder auf das daran angekoppelte Testelement zum Ausführen einer Probenaufnahmebewegung übertragen wird.

Ein analytisches Hilfsmittel ist in diesem Zusammenhang ein Mittel, das die zwei Funktionen Einstechen einer Lanzette und Probenaufnahme, insbesondere die drei Funktionen Einstechen, Probenaufnahme und Bereitstellen einer Testschemie zur Analyse der Probe zusammenfasst. Für die vorliegende Erfindung können zum Beispiele einzelne streifenförmige analytische Hilfsmittel oder eine Vielzahl von auf einem Band angeordneten analytischen Hilfsmittel zum Einsatz kommen. Gemäß einer bevorzugten Ausführungsform der vorliegenden Erfindung wird eine Vielzahl von analytischen Hilfsmitteln auf einem Analyseband verwendet.

Jedes für die vorliegende Erfindung verwendete analytische Hilfsmittel enthält eine Lanzette und ein Testelement. Das Testelement weist ein Testfeld zur Analyse der flüssigen Probe auf. Ein Testfeld ist in diesem Zusammenhang ein begrenzter Bereich des Testelements, in dem sich die flüssige Probe während ihrer z. B. elektrochemisch oder photometrisch durchgeführten Analyse befindet. Das Testfeld kann eine Nachweischemie enthalten, die mit der Probe reagiert und dadurch einen bei der Analyse nachweisbaren Effekt, insbesondere in Abhängigkeit von der Konzentration eines Analyten in der Probe, verursacht (z. B. eine Farbänderung). In einem solchen Testfeld kann beispielsweise die Konzentration von Glukose in einer Körperflüssigkeit wie Blut oder interstitieller Flüssigkeit analysiert werden.

Das erfindungsgemäße Probengewinnungssystem enthält ein Kopplungselement, das nacheinander in einer ersten Position eines analytischen Hilfsmittels an die Lanzette und in einer zweiten Position des analytischen Hilfsmittels an das Testelement koppelbar ist. Das analytische Hilfsmittel kann dazu automatisch oder manuell aus der ersten in die zweite Position transportiert werden. Vorzugsweise umfasst das erfindungsgemäße Probengewinnungssystem zum automatischen Transport eine Transporteinrichtung, um das analytische Hilfsmittel relativ zu dem in Ruheposition angeordneten Kopplungselement aus der ersten Position in die zweite Position zu transportieren. Falls das analytische Hilfsmittel auf einem Analyseband angeordnet ist, das in einer Kassette auf Rollen aufgerollt ist, so kann eine solche Transportvorrichtung, z. B. eine Rolle zum Aufrollen des Analysebandes, um einen bestimmten Drehwinkel rotieren, bis sich das nächste Testelement oder die nächste Lanzette in der gewünschten Position relativ zu dem Kopplungselement befindet.

Bei der vorliegenden Erfindung wird das Kopplungselement nacheinander an die Lanzette gekoppelt und an das Testelement gekoppelt. Wenn das Kopplungselement an die Lanzette gekoppelt ist, bewirkt seine definierte Bewegung aus einer Ruheposition (unausgelenkten Position) in seine ausgelenkte Position, dass die Lanzette ebenfalls eine Bewegung ausführt, nämlich eine Stechbewegung durchführt. Wenn das Kopplungselement an das Testelement gekoppelt ist, bewirkt seine definierte Bewegung aus der Ruheposition in die ausgelenkte Position, dass das Testelement ebenfalls eine Bewegung ausführt, nämlich eine Probenaufnahmebewegung durchführt.

Das Koppeln der Lanzette oder des Testelements an das Kopplungselement kann bei der vorliegenden Erfindung in einer beliebigen, dem Fachmann bekannten Art und Weise erfolgen, die sicherstellt, dass eine Bewegung des Kopplungselements aus seiner Ruheposition in seine ausgelenkte Position zu einer Stechbewegung der Lanzette beziehungsweise zu einer Probenaufnahmebewegung des Testelements führt. Die Kopplung kann dabei direkt oder indirekt über weitere Bauteile erfolgen. Beispielsweise kann das Kopplungselement das analytische Hilfsmittel an der jeweiligen Position umgreifen und bei seiner Bewegung in die ausgelenkte Position um einen definierten Weg anheben. Die Kopplung kann ferner eine aktive Kopplung oder eine passive Kopplung sein. Bei einer aktiven Kopplung koppelt das Kopplungselement formschlüssig an die Lanzette oder das Testelement (z.B. durch ein Greifen der Lanzette oder des Testelements), so dass auch die Rückbewegung der Lanzette oder des Testelements aus der ausgelenkten Position in die Ruheposition durch das Kopplungselement geführt und angetrieben wird. Bei einer passiven Kopplung schiebt oder stößt das Kopplungselement die Lanzette oder das Testelement in die ausgelenkte Position. Die Rückbewegung der Lanzette oder des Testelements in die Ruheposition muss dann durch ein zusätzliches Antriebselement erfolgen, beispielsweise durch eine Feder, die bei der Auslenkung der Lanzette oder des Testelements gespannt wird und bei ihrer Entspannung auf die Lanzette oder das Testelement wirkt, so dass eine Rückbewegung der Lanzette oder des Testelements in die Ruheposition erfolgt.

Das erfindungsgemäße Probengewinnungssystem enthält ferner eine Antriebseinheit, die eine Bewegung des Kopplungselements aus der Ruheposition in die ausgelenkte Position antreibt. Die Antriebseinheit stellt insbesondere die Energie zum Bewegen des Kopplungselements und Mittel zum Übertragen der Energie auf das Kopplungselement bereit.

Die durch die Antriebseinheit angetriebene Bewegung des Kopplungselements aus der Ruheposition in die ausgelenkte Position und wieder zurück in die Ruheposition wird erfindungsgemäß auf die daran angekoppelte Lanzette zum Ausführen einer Stechbewegung oder auf das daran angekoppelte Testelement zum Ausführen einer Probenaufnahmebewegung übertragen.

Die Stechbewegung ist in diesem Zusammenhang eine geführte Bewegung, bei der die Lanzette um einen bestimmten Weg vor- und anschließend wieder zurückbewegt wird. Dabei kann die Spitze der Lanzette z. B. aus einer Öffnung in einem Gehäuse des erfindungsgemäßen Probengewinnungssystems um eine definierte Länge austreten, wobei diese Länge die Einstichtiefe bestimmt, und z. B. in die Haut einer Fingerbeere eines Patienten einstechen, um eine Perforation der Haut zu erzeugen. Die Geschwindigkeit der Stechbewegung wird dabei vorzugsweise so gesteuert, dass ein schmerzarmer Einstich gewährleistet wird.

Eine Probenaufnahmebewegung ist in diesem Zusammenhang eine Bewegung, bei der das Testelement insbesondere ein auf einem Analyseband enthaltenes Testelement, um einen bestimmten Weg vor- und anschließend wieder zurückbewegt wird. Diese Bewegung ist vorzugsweise langsamer als die Stechbewegung. Bei der Probenaufnahmebewegung kann ein Probenaufnahmeort (z. B. ein Eingang einer Kapillare zum Probentransport zu dem Testfeld des Testelements oder das Testfeld selbst) aus einer dafür vorgesehenen Öffnung in einem Gehäuse des erfindungsgemäßen Probengewinnungssystems um eine bestimmte Wegstrecke austreten, so dass die Probe mit dem Probenaufnahmeort in Berührung kommt und auf diesen übertragen werden kann.

Die Erfindung bezieht sich weiterhin auf ein Verfahren zum Gewinnen einer flüssigen Probe in einem Probengewinnungssystem mittels mindestens eines analytischen Hilfsmittels,
wobei das analytische Hilfsmittel eine Lanzette und ein Testelement enthält und das Testelement ein Testfeld zur Analyse der flüssigen Probe aufweist. Bei dem Verfahren wird ein Kopplungselement des Probengewinnungssystems nacheinander in einer ersten Position des analytischen Hilfsmittels an die Lanzette und in einer zweiten Position des analytischen Hilfsmittels an das Testelement gekoppelt. Eine Antriebseinheit treibt eine Bewegung des Kopplungselements aus einer Ruheposition in eine ausgelenkte Position an, so dass die Bewegung des Kopplungselements auf die daran angekoppelte Lanzette zum Ausführen einer Stechbewegung oder auf das daran angekoppelte Testelement zum Ausführen einer Probenaufnahmebewegung übertragen wird.

Die vorliegende Erfindung ermöglicht einen einfachen und kostengünstigen Aufbau eines Probengewinnungssystems. Durch die Verwendung desselben Kopplungselements zum Bewegen der Lanzette (Ausführen des Stechhubes) und anschließend des Testelements (Sammeln einer zu analysierenden Probe) wird der Bauraum des erfindungsgemäßen Probengewinnungssystems gering gehalten.

Gemäß einer bevorzugten Ausführungsform der vorliegenden Erfindung enthält die Antriebseinheit einen mechanischen Energiespeicher, der seine Energie an das Kopplungselement zur Bewegung in die ausgelenkte Position abgeben kann, und gegebenenfalls einen Motor zum Aufladen des mechanischen Energiespeichers. Um ein möglichst schmerzarmes Einstechen zu erreichen, sollte die Lanzette eine große Beschleunigung erfahren, so dass die Lanzette bei der Stechbewegung mit hoher Geschwindigkeit in das Körperteil einsticht. Dazu ist vorzugsweise der mechanische Energiespeicher vorgesehen, dessen gespeicherte Energie zumindest teilweise in kinetische Energie der Lanzette umgewandelt werden kann. Nach dem Ausführen der Stechbewegung ist der Energiespeicher weitgehend oder vollständig entladen. Ein Motor kann dann den mechanischen Energiespeicher mit Energie für die nächste Stechbewegung versorgen. Das Aufladen kann jedoch auch manuell erfolgen. Ein Beispiel für einen bevorzugten mechanischen Energiespeicher ist eine Feder, die zum Aufladen z.B. durch den Motor oder manuell durch einen Patienten expandiert oder komprimiert wird und sich zur Energieübertragung auf die Lanzette entspannt. Ein mechanischer Energiespeicher zeichnet sich durch eine hohe Entnahmegeschwindigkeit aus.

Der gegebenenfalls zum Aufladen des mechanischen Energiespeichers vorgesehene Motor ist vorzugsweise ein elektrischer Motor (insbesondere ein DC-Motor, bürstenloser Außenläufermotor) oder ein Formgedächtnislegierungsantrieb ("Shape Memory Alloy Actuator").

Vorzugsweise umfasst die Antriebseinheit des erfindungsgemäßen Probengewinnungssystems einen mechanischen Bewegungswandler, wobei das Kopplungselement über den mechanischen Bewegungswandler an den mechanischen Energiespeicher gekoppelt ist. Der mechanische Bewegungswandler ist ein Mittel, das unter anderem die durch den Energiespeicher freigesetzte Energie mechanisch in die gewünschte Bewegung des Kopplungselements umsetzt, die zu der Stechbewegung der Lanzette führt. Der mechanische Bewegungswandler überträgt Energie des mechanischen Energiespeichers auf das Kopplungselement, was zu einer Bewegung des Kopplungselements aus seiner Ruheposition in die ausgelenkte Position und wieder zurück in die Ruheposition führt, so dass die an das Kopplungselement gekoppelte Lanzette eine Stechbewegung ausführt. Vorzugsweise ist die Antriebseinheit des erfindungsgemäßen Probengewinnungssystems und besonders bevorzugt der mechanische Bewegungswandler so gestaltet, dass die Antriebseinheit beziehungsweise der mechanische Bewegungswandler während des Aufladens des mechanischen Energiespeichers Energie (z.B. des Motors) auf das Kopplungselement zum Ausführen der Probenaufnahmebewegung durch das Testelement überträgt. Insbesondere kann der mechanische Bewegungswandler eine Ladebewegung, die z.B. der Motor oder ein Patient manuell zum Aufladen des Energiespeichers antreibt (beispielsweise eine Spannbewegung zum Spannen einer Feder), gleichzeitig auf das Kopplungselement zum Ausführen der Probenaufnahmebewegung durch das Testelement übertragen. Somit wird für die Stechbewegung beziehungsweise für die Vorbereitung der Stechbewegung und für die Probenaufnahmebewegung nur ein einziger Motor oder ein einziger Handhabungsschritt des Patienten benötigt, was sich wiederum platzsparend auf das erfindungsgemäße Probengewinnungssystem auswirkt.

Eine mögliche Ausführungsform dieser Variante ist ein Elektromotor, der zum Spannen einer als mechanischer Energiespeicher dienenden Feder vorgesehen ist. Die durch die gespannte Feder freigesetzte Energie wird über den mechanischen Bewegungswandler auf das mit der Lanzette gekoppelte Kopplungselement übertragen. Das Kopplungselement wird dadurch aus der Ruheposition in die ausgelenkte Position und zurück bewegt, wodurch die Lanzette die Stechbewegung ausführt. Anschließend wird das analytische Hilfsmittel aus der ersten in die zweite Position transportiert, in der das Testelement an das Kopplungselement gekoppelt wird. Dann spannt der Motor die Feder (Aufladen des mechanischen Energiespeichers), wobei gleichzeitig die durch den Motor angetriebene Spannbewegung durch den mechanischen Bewegungswandler auf das mit dem Testelement gekoppelte Kopplungselement übertragen wird. Das Kopplungselement wird dadurch aus der Ruheposition in die ausgelenkte Position und zurück bewegt, wodurch das Testelement die Probenaufnahmebewegung ausführt. Anschließend ist die Feder erneut gespannt und bereit zum Antreiben einer weiteren Stechbewegung. Diese bevorzugte Ausführungsform der Erfindung basiert folglich darauf, dass die Bewegung zum Aufladen des mechanischen Energiespeichers (z. B. das Spannen einer Feder) als nutzbare Bewegung eingesetzt wird, nämlich zum Ausführen einer Probenaufnahmebewegung durch das Testelement.

Die Erfindung bezieht sich weiterhin auf ein Verfahren zum Gewinnen einer flüssigen Probe in einem Probengewinnungssystem, mit den Verfahrensschritten:
- Aufnehmen der flüssigen Probe auf ein analytisches Hilfsmittel, das eine Lanzette und ein Testelement mit einem Testfeld zur Analyse der flüssigen Probe umfasst, durch Antreiben eines an das Testelement gekoppelten Kopplungselements (z.B. manuell oder mittels eines Motors) zum Ausführen einer Probenaufnahmebewegung durch das Testelement und
- gleichzeitiges Aufladen eines mechanischen Energiespeichers (z.B. manuell oder durch den Motor) mit Energie zum Antreiben einer Stechbewegung der an das Kopplungselement gekoppelten Lanzette,
wobei das nacheinander an die Lanzette und das Testelement gekoppelte Kopplungselement sowohl bei der Stechbewegung als auch bei der Probenaufnahmebewegung aus einer Ruheposition in eine ausgelenkte Position und zurück bewegt wird.

Gemäß einer bevorzugten Ausführungsform der vorliegenden Erfindung weist der mechanische Bewegungswandler eine Kulisse mit einer Kulissenführung auf, in die ein an dem Kopplungselement angeordnetes Eingriffselement eingreift. Dabei sind der mechanische Energiespeicher und gegebenenfalls ein Motor vorzugsweise über ein Antriebselement an den Kulissenkörper gekoppelt, so dass Energie des mechanischen Energiespeichers auf den Kulissenkörper zur Bewegung des Kulissenkörpers in einer ersten Richtung relativ zu dem Kopplungselement übertragen wird und Energie (z.B. des Motors) während des Aufladens des mechanischen Energiespeichers auf den Kulissenkörper zur Bewegung des Kulissenkörpers an einer zweiten, der ersten entgegengesetzten Richtung relativ zu dem Kopplungselement übertragen werden kann. Der Kulissenkörper ist folglich durch den Energiespeicher in einer Richtung und z.B. manuell oder durch den Motor in der anderen Richtung verschiebbar. Das Kopplungselement ist hingegen entlang der Verschieberichtung des Kulissenkörpers ortsfest positioniert, jedoch senkrecht zu der Verschieberichtung aus der Ruheposition in die ausgelenkte Position bewegbar. Beim Verschieben des Kulissenkörpers wird das Eingriffselement des Kopplungselements entlang der Kulissenführung geführt, die als Steuerkurve eine Auslenkung des Kopplungselements senkrecht zur Bewegung des Kulissenkörpers bedingt. Die Kulissenführung ist dabei symmetrisch mit einem zwischen zwei Minima angeordneten Maximum ausgebildet, so dass das Kopplungselement sowohl bei der Hin- als auch bei der Rückbewegung des Kulissenkörpers einen Bewegungshub aus der Ruheposition in die maximal ausgelenkte Position und zurück in die Ruheposition erfährt. Die Hinbewegung des Kulissenkörpers wird dabei z. B. durch eine Feder angetrieben und die Rückbewegung z.B. durch einen elektrischen Motor beim erneuten Spannen der Feder angetrieben. Nach der Ausführung der Hinbewegung und vor der Ausführung der Rückbewegung wird dabei die Lanzette von dem Kopplungselement abgekoppelt und das Testelement, insbesondere ein auf einem Analyseband enthaltenes Testelement, an das Kopplungselement gekoppelt.

Gemäß einer anderen bevorzugten Ausführungsform der vorliegenden Erfindung ist der mechanische Bewegungswandler als Pleuelantrieb aufgebaut, wobei der Pleuelantrieb eine Pleuelstange und einen Antriebsrotor aufweist, die so mit dem Kopplungselement zusammenwirken, dass eine rotierende Bewegung des Antriebsrotors in eine lineare Bewegung des Kopplungselements umgesetzt werden kann.

Der Antriebsrotor (z. B. eine Antriebswelle) ist folglich durch den mechanischen Energiespeicher (z. B. eine Spiralfeder) in einer Richtung und (z.B. durch einen Motor) in der anderen Richtung drehbar. Beim Drehen des Antriebsrotors wird die Drehbewegung durch die Pleuelstange in eine lineare Bewegung des Kopplungselements umgesetzt. Der Drehwinkel wird dabei so gewählt, dass das Kopplungselement sowohl bei der Hindrehung als auch bei der Rückdrehung des Antriebsrotors einen Bewegungshub aus der Ruheposition in die maximal ausgelenkte Position und wieder zurück erfährt. Die Hindrehung wird dabei z. B. durch eine Spiralfeder angetrieben und die Rückdrehung z.B. durch einen elektrischen Motor oder manuell beim erneuten Spannen der Spiralfeder angetrieben. Nach der Ausführung der Hindrehung und vor der Ausführung der Rückdrehung wird dabei die Lanzette von dem Kopplungselement abgekoppelt und das Testelement an das Kopplungselement angekoppelt oder zumindest auf das Ankoppeln vorbereitet.

Die Antriebseinheit des erfindungsgemäßen Probengewinnungssystems enthält vorzugsweise ein Antriebselement, das zumindest die drei folgenden Funktionen übernimmt:
1. Übertragen von Energie des Energiespeichers als kinetische Energie auf das Kopplungselement und damit auf die daran koppelbare Lanzette zum Ausführen einer Stechbewegung,
2. Übertragen von Energie des Motors oder manuell durch einen Benutzer eingebrachte Energie als kinetische Energie auf das Kopplungselement und damit auf das daran koppelbare Testelement, insbesondere ein auf einem Analyseband enthaltenes Testelement, zum Ausführen einer Probenaufnahmebewegung und
3. Übertragen von Energie des Motors oder manuell durch einen Benutzer eingebrachte Energie auf den mechanischen Energiespeicher zum Aufladen des mechanischen Energiespeichers.

Als ein solches Antriebselement kann bei der Ausführungsvariante mit Kulisse z. B. ein Mitnehmer dienen, der bei der Expansion einer Feder (mechanischer Energiespeicher) in eine Richtung und beim Antrieb manuell durch einen Benutzer oder durch den Motor in die andere Richtung bewegt wird und der bei der Bewegung manuell durch einen Benutzer oder durch den Motor die Feder spannt und bei beiden Bewegungen (durch die Feder und den Benutzer/Motor) den Kulissenkörper mitnimmt.

Bei der Ausführungsvariante mit Pleuelantrieb kann als ein solches Antriebselement ein Antriebsrotor dienen, der bei der Entspannung einer Feder (mechanischer Energiespeicher) in einer Drehrichtung und beim Antrieb manuell durch einen Benutzer oder durch den Motor in der anderen Drehrichtung gedreht wird und der bei der Drehung manuell durch einen Benutzer oder durch den Motor die Feder spannt und bei beiden Drehungen (durch die Feder und den Motor) die Pleuelstange und damit das Kopplungselement bewegt.

Die Erfindung bezieht sich weiterhin auf ein Probengewinnungssystem zum Gewinnen einer flüssigen Probe, enthaltend eine Lanzette, ein Gehäuse und eine Antriebseinheit zum Antreiben der Lanzette, so dass die Lanzette zum Ausführen einer Stechbewegung zumindest teilweise aus dem Gehäuse austreten kann. Die Antriebseinheit enthält einen mechanischen Energiespeicher, der seine Energie an die Lanzette zum Ausführen einer Stechbewegung abgeben kann, und gegebenenfalls einen Motor zum Aufladen des mechanischen Energiespeichers. Die Antriebseinheit umfasst einen mechanischen Bewegungswandler,
wobei die Lanzette über den mechanischen Bewegungswandler an den mechanischen Energiespeicher gekoppelt ist und der mechanische Bewegungswandler einen Pleuelantrieb umfasst. Der Pleuelantrieb weist eine Pleuelstange und einen Antriebsrotor auf, die so mit der Lanzette zusammenwirken, dass eine rotierende Bewegung des Antriebsrotors in eine lineare Bewegung der Lanzette umgesetzt werden kann.

Zusätzlich kann der mechanische Bewegungswandler oder der Motor an eine weitere, von dem mechanischen Energiespeicher unabhängige Systemfunktion des Probengewinnungssystems oder eines das Probengewinnungssystem enthaltenden Analysesystems gekoppelt sein. Die von dem mechanischen Energiespeicher unabhängige Systemfunktion kann z.B. mindestens eine der Funktionen ausgewählt aus der Gruppe Ausführen der Probenaufnahmebewegung des Testelements, Transport eines die Lanzette aufweisenden analytischen Hilfsmittels, Testelementtransport und Testelementmagazintransport sein. Gemäß dieser Ausführungsform wird z.B. der Motor als kombinierter Antrieb verwendet. Bei dem kombinierten Antrieb lädt der (vorzugsweise elektrisch betriebene) Motor zum einen den mechanischen Energiespeicher auf und treibt gleichzeitig zeitlich versetzt dazu oder zeitlich unabhängig davon eine weitere Systemfunktion an. Bei dem oben beschriebenen erfindungsgemäßen Probengewinnungssystem ist dies die Systemfunktion des Ausführens der Probenaufnahmebewegung durch das Testelement.

Die Erfindung bezieht sich weiterhin auf ein Probengewinnungssystem zum Gewinnen einer flüssigen Probe, enthaltend mindestens ein analytisches Hilfsmittel, das eine Lanzette und ein Testelement aufweist, und einen mechanischen Energiespeicher (z.B. eine Feder), der Energie an die Lanzette zum Ausführen einer Stechbewegung abgeben kann. Das Probengewinnungssystem umfasst Übertragungsmittel, die so an den mechanischen Energiespeicher und das analytische Hilfsmittel gekoppelt sind, dass eine Bewegung der Übertragungsmittel gleichzeitig Energie auf den mechanischen Energiespeicher zum Aufladen des mechanischen Energiespeichers überträgt und eine Bewegung des Testelements antreibt. Dieses Probengewinnungssystem kann dabei eine Vielzahl von Merkmalen der bisher beschriebenen Probengewinnungssysteme aufweisen.

Die Erfindung bezieht sich auch auf ein Verfahren zum Gewinnen einer flüssigen Probe in einem Probengewinnungssystem mittels mindestens eines analytischen Hilfsmittels, wobei das analytische Hilfsmittel eine Lanzette und ein Testelement enthält. Eine Bewegung von Übertragungsmitteln des Probengewinnungssystems, die zum Aufladen eines mechanischen Energiespeichers mit Energie zum Antreiben einer Stechbewegung der Lanzette dient, wird dabei gleichzeitig genutzt, um eine Bewegung des Testelements anzutreiben

Die Übertragungsmittel gemäß der vorliegenden Erfindung sind Mittel, die zum Übertragen von Energie auf den mechanischen Energiespeicher dienen, um den mechanischen Energiespeicher aufzuladen. Beispielsweise sind dies Mittel, die zum Spannen einer als mechanischer Energiespeicher vorgesehenen Feder dienen. Der aufgeladene mechanische Energiespeicher kann anschließend Energie abgeben, um eine Stechbewegung der Lanzette des analytischen Hilfsmittels anzutreiben. Falls der mechanische Energiespeicher z.B. eine Feder ist, kann diese durch ihre Entspannung Energie an die Lanzette zum Ausführen der Stechbewegung abgeben.

Zum Aufladen des mechanischen Energiespeichers wird zumindest ein Bestandteil der Übertragungsmittel bewegt. Der Bestandteil führt z.B. eine lineare Translationsbewegung oder eine Rotationsbewegung aus. Durch diese Bewegung wird Energie auf den mechanischen Energiespeicher übertragen (beispielsweise wird eine Feder gespannt). Die Bewegung des Bestandteils der Übertragungsmittel führt zusätzlich und vorzugsweise unabhängig von dem Aufladen des mechanischen Energiespeichers zu einer Bewegung des Testelements, beispielsweise zu einer Probenaufnahmebewegung, durch die eine flüssige Probe auf eine Testfeld des Testelements aufgenommen wird. Beispielsweise folgt aus der Bewegung des Bestandteils der Übertragungsmittel eine Bewegung eines weiteren Bestandteils der Übertragungsmittel, die die Bewegung des Testelements antreibt.

Die Erfindung bezieht sich auch auf ein Verfahren zum Gewinnen einer flüssigen Probe in einem Probengewinnungssystem mittels mindestens eines analytischen Hilfsmittels, wobei das analytische Hilfsmittel eine Lanzette und ein Testelement enthält. Eine Bewegung von Übertragungsmitteln des Probengewinnungssystems, die zum Aufladen eines mechanischen Energiespeichers mit Energie zum Antreiben einer Stechbewegung der Lanzette dient, wird dabei gleichzeitig genutzt, um eine Bewegung des Testelements anzutreiben

Die Übertragungsmittel können bei dem erfindungsgemäßen Probengewinnungssystem und/oder bei dem erfindungsgemäßen Verfahren beispielsweise einen Pleuelantrieb oder einen Kulissenkörper mit einer Kulissenführung umfassen.

Bei einem Pleuelantrieb kann z.B. eine Rotationsbewegung des Antriebsrotors zum Spannen einer Feder dienen (Aufladen des mechanischen Energiespeichers). Der Antriebsrotor kann z.B. durch einen Motor oder manuell durch einen Benutzer rotiert werden. Durch die Rotationsbewegung des Antriebsrotors wird die an dem Antriebsrotor montierte Pleuelstange bewegt. Diese Bewegung der Pleuelstange treibt wiederum (unabhängig vom Spannen der Feder) eine Bewegung (z.B. eine Probenaufnahmebewegung) des Testelements an.

Bei einer Kulisse wird z.B. eine Translationsbewegung des Kulissenkörpers zum Spannen einer Feder (Aufladen des mechanischen Energiespeichers) genutzt. Der Kulissenkörper kann dazu z.B. durch einen Motor oder manuell durch einen Benutzer verschoben werden. Durch die Translationsbewegung des Kulissenkörpers wird die in diesem vorhandene Kulissenführung mitbewegt und damit auch ein in die Kulissenführung eingreifendes Eingriffselement. Diese Bewegung des Eingriffselements (geführt durch die Kulissenführung) treibt wiederum (unabhängig vom Spannen der Feder) eine Bewegung (z.B. eine Probenaufnahmebewegung) des Testelements an.

Ferner können die Übertragungsmittel durch eine erste Bewegung Energie des mechanischen Energiespeichers auf die Lanzette zum Ausführen der Stechbewegung übertragen und durch eine zweite Bewegung gleichzeitig Energie zum Aufladen des mechanischen Energiespeichers übertragen und die Bewegung des Testelements antreiben.

Bei einem Pleuelantrieb kann z.B. eine Rotationsbewegung des Antriebsrotors in einer Richtung zum Spannen einer Feder dienen (Aufladen des mechanischen Energiespeichers) und eine Rotationsbewegung des Antriebsrotors in der anderen Richtung (beim Entspannen der Feder - Entladen des mechanischen Energiespeichers) über die Pleuelstange auf die Lanzette zum Ausführen der Stechbewegung übertragen werden.

Bei einer Kulisse wird z.B. eine Translationsbewegung des Kulissenkörpers in einer Richtung zum Spannen einer Feder (Aufladen des mechanischen Energiespeichers) genutzt und eine Translationsbewegung des Kulissenkörpers in der anderen Richtung (beim Entspannen der Feder - Entladen des mechanischen Energiespeichers) über die Kulissenführung und das Eingriffselement auf die Lanzette zum Ausführen der Stechbewegung übertragen.

Die Erfindung bezieht sich weiterhin auf ein Analysesystem zum Analysieren einer flüssigen Probe, das ein erfindungsgemäßes Probengewinnungssystem und eine Messanordnung umfasst.

Das Analysesystem kann zur Durchführung einer elektrochemischen und/oder photometrischen Analyse ausgelegt sein.

Bei photometrischen Analysesystemen enthalten die Testelemente ein Reagenzsystem, dessen Reaktion mit dem Analyten zu einer photometrisch nachweisbaren Veränderung (einem Farbumschlag) führt. Die Reagenzien befinden sich dabei üblicherweise in einem Testfeld des Testelements, wobei deren Farbe sich in Abhängigkeit von der Konzentration ändert. Diese Farbänderung lässt sich mit Hilfe einer Messanordnung quantitativ durch Reflexionsphotometrie bestimmen.

Elektrochemische Testelemente enthalten ein elektrochemisches Reagenzsystem, dessen Reaktion mit dem Analyten die zwischen zwei Polen des Testelements anliegende elektrische Spannung und/oder die zwischen zwei Polen des Testelements bei definierter Spannung fließende Stromstärke beeinflusst. In diesem Fall ist also die Spannung oder Stromstärke die physikalisch messbare Größe, die mit einer entsprechenden in dem Analysesystem integrierten, als Spannungs- oder Strommesseinrichtung gestalteten Messanordnung bestimmt und deren mit der Konzentration des Analyten korrelierende Änderung in die Analysedaten (Konzentration des Analyten) umgerechnet wird.

Die Erfindung bezieht sich ferner auf die Verwendung eines erfindungsgemäßen Probengewinnungssystems zur Gewinnung einer Blutprobe und auf die Verwendung eines erfindungsgemäßen Analysesystems zum Analysieren des Glukosegehalts in einer Körperflüssigkeit.

Anhand der Zeichnung wird die Erfindung nachstehend näher erläutert.

Es zeigen
- Figuren 1A bis 1F: den Bewegungsablauf bei einer schematisch dargestellten ersten Ausführungsform eines erfindungsgemäßen Probengewinnungssystems mit Kulisse und
- Figuren 2A bis 2F: den Bewegungsablauf bei einer schematisch dargestellten zweiten Ausführungsform eines erfindungsgemäßen Probengewinnungssystems mit Pleuelantrieb.

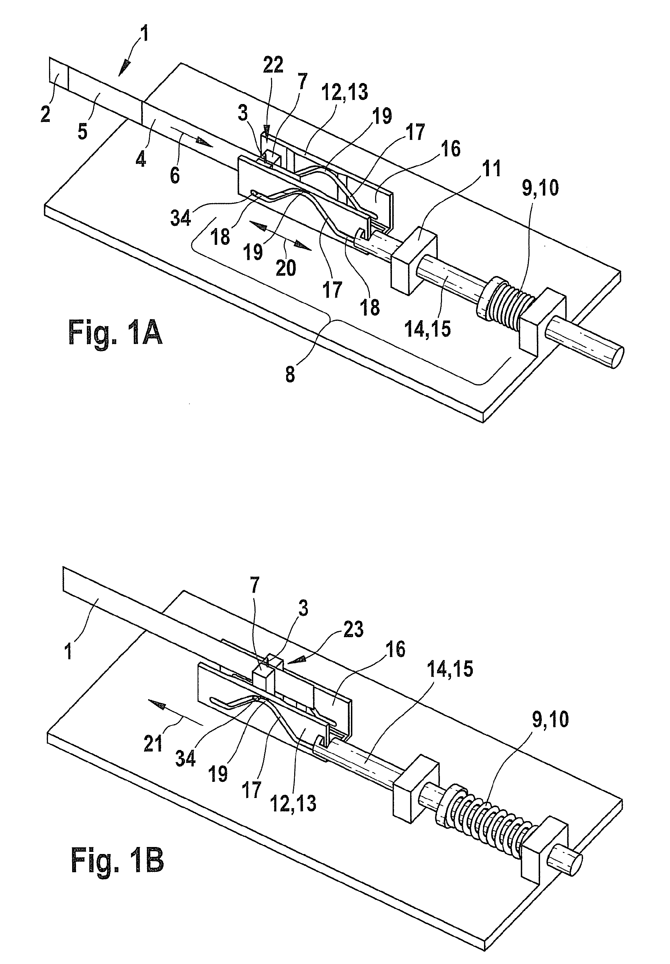

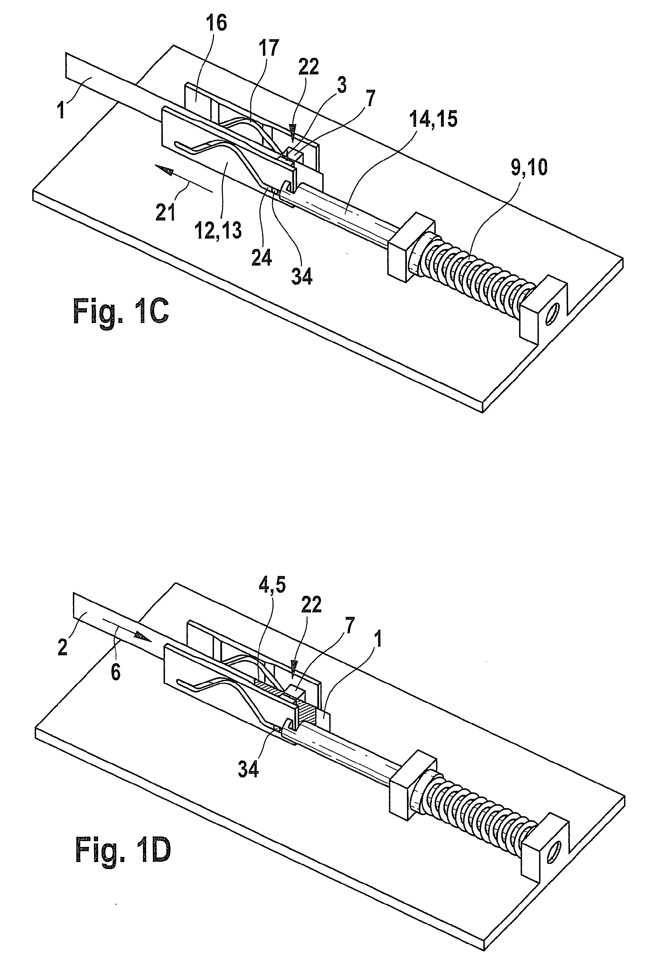

Figur 1A zeigt schematisch eine erste Ausführungsform eines erfindungsgemäßen Probengewinnungssystems im Ausgangszustand.

Das Probengewinnungssystem enthält ein analytisches Hilfsmittel 1, wobei es sich um ein auf einem Analyseband 2 angeordnetes analytisches Hilfsmittel 1 handelt. Von dem Analyseband 2 ist lediglich ein Abschnitt dargestellt. Das analytische Hilfsmittel 1 enthält eine Lanzette 3 und ein Testelement 4. Das Testelement 4 weist ein Testfeld 5 zur Analyse einer flüssigen Probe, insbesondere zur Analyse einer Blutprobe auf. Das Analyseband 2 ist in der Transportrichtung 6 mittels einer (nicht dargestellten) Transportvorrichtung weiterbewegbar. Die Lanzette 3 ist senkrecht zu der Transportrichtung 6 auf dem analytischen Hilfsmittel 1 angeordnet.

Das Probengewinnungssystem enthält ein Kopplungselement 7 in Form eines Greifers, das in Figur 1A an die Lanzette angekoppelt ist (erste Position des analytischen Hilfsmittels 1). Das Probengewinnungssystem enthält ferner eine Antriebseinheit 8, um eine Bewegung des Kopplungselements 7 anzutreiben. Die Antriebseinheit 8 umfasst eine Feder 9 als mechanischen Energiespeicher 10, einen Motor 11, eine Kulisse 12 als mechanischen Bewegungswandler 13 und einen Mitnehmer 14, der als Antriebselement 15 den mechanischen Energiespeicher 10, den Motor 11 und den Kulissenkörper 16 der Kulisse 12 miteinander koppelt. Die Feder 9 ist gespannt (aufgeladener mechanischer Energiespeicher 10). Die Kulisse 12 umfasst den Kulissenkörper 16, der eine Kulissenführung 17 aufweist. Die Kulissenführung 17 hat die Form eines zwischen zwei Minima 18 angeordneten Maximums 19 und ist symmetrisch aufgebaut. In die Kulissenführung 17 greift ein Eingriffselement 34 des Kopplungselements 7 ein. Das Kopplungselement 7 ist in Bezug auf die mögliche Bewegungsrichtung 20 des Kulissenkörpers 16 ortsfest angeordnet. Es kann durch die Kulissenführung 17, in die sein Eingriffselement 34 eingreift, lediglich senkrecht zu dieser Bewegungsrichtung 20 bewegt werden. In Figur 1A ist das Kopplungselement 7 in seiner Ruheposition 22 dargestellt.

Figur 1B zeigt das erfindungsgemäße Probengewinnungssystem aus Figur 1A nach dem Auslösen des Stechvorgangs.

Durch das Auslösen entspannt sich die gespannte Feder 9 und der mechanische Energiespeicher 10 gibt seine gespeicherte Energie zumindest teilweise über das Antriebselement 15 als kinetische Energie an den Kulissenkörper 16 ab. Der Kulissenkörper 16 wird dadurch in einer ersten Richtung 21 relativ zu dem Kopplungselement 7 bewegt. Dabei läuft das Eingriffselement 34 des Kopplungselements 7 die Kulissenführung 17 ab, wodurch das Kopplungselement 7 aus der Ruheposition in die in Figur 1B dargestellte ausgelenkte Position 23 angehoben wird. Das Kopplungselement 7 hat das Maximum 19 der Kulissenführung 17 erreicht. Die an das Kopplungselement 7 angekoppelte Lanzette 3 wird dadurch ebenfalls angehoben und sticht durch diese Bewegung z. B. in ein Körperteil eines Patienten, um eine Öffnung in der Haut zu erzeugen und eine Blutprobe zu gewinnen.

Figur 1C zeigt das erfindungsgemäße Probengewinnungssystem aus Figuren 1A und 1B nach dem Ausführen einer Stechbewegung.

Nachdem das Kopplungselement 7 das Maximum 19 der Kulissenführung 17 erreicht hat, bewegt sich der Kulissenkörper 16 durch das weitere Entspannen der Feder 9 noch weiter in der ersten Richtung 21 relativ zu dem Kopplungselement 7. Das Eingriffselement 34 des Kopplungselements 7 fährt dabei weiterhin die Kulissenführung 17 ab, bis die Feder 9 vollständig entspannt ist. In diesem Zustand befindet sich das Kopplungselement 7 auf der Höhe des zweiten Minimums 24 der Kulissenführung 17, so dass es sich wieder in seiner Ruheposition 22 befindet. Die Lanzette 3 ist nun wieder zurückgezogen und hat somit ihre Stechbewegung abgeschlossen.

Figur 1D zeigt das erfindungsgemäße Probengewinnungssystem aus Figuren 1A bis 1C, nachdem das analytische Hilfsmittel weiter transportiert wurde.

Eine (nicht dargestellte) Transporteinrichtung transportiert das analytische Hilfsmittel 1 nach der erfolgten Stechbewegung der Lanzette 3 in Transportrichtung 6 relativ zu dem in Ruheposition 22 angeordneten Kopplungselement 7 aus der ersten in die zweite Position, in der das Kopplungselement 7 an das Testelement 4 gekoppelt ist. Dazu wird das Analyseband 2, das das analytische Hilfsmittel 1 trägt, in Transportrichtung 6 vorgespult, bis die gewünschte zweite Position zum Ankoppeln des Testelements 4 an das Kopplungselement 7 erreicht wird.

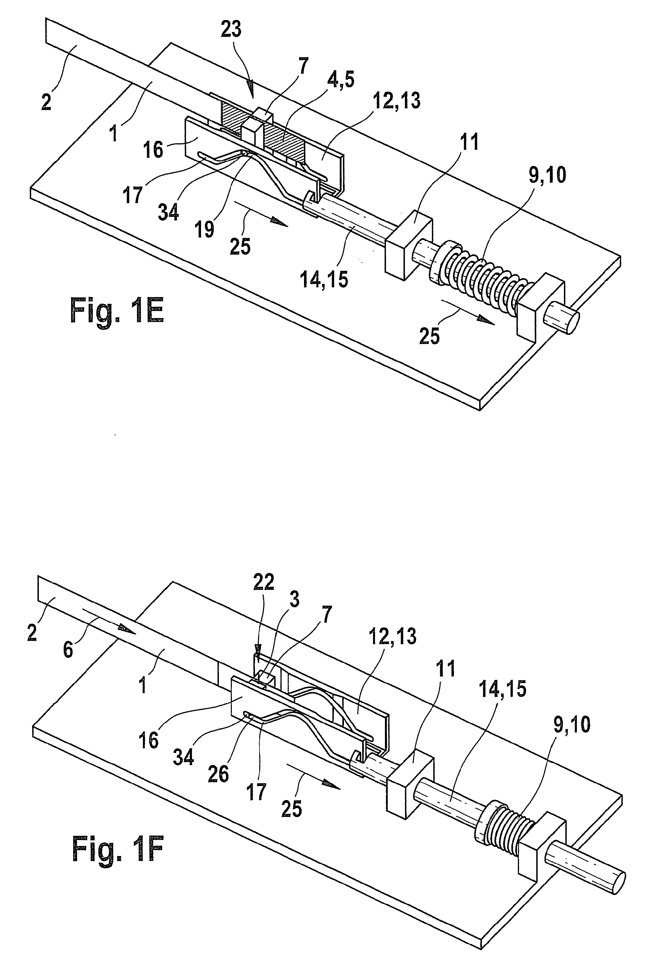

Figur 1E zeigt das erfindungsgemäße Probengewinnungssystem aus Figuren 1A bis 1D beim Ausführen der Probenaufnahmebewegung durch das Testelement.

Das Testelement 4 ist an das Kopplungselement 7 gekoppelt. Der Motor 11 bewegt das Antriebselement 15 in der zweiten Richtung 25, die der ersten Richtung 21 entgegengesetzt ist. Dadurch wird der an das Antriebselement 15 gekoppelte Kulissenkörper 16 in der zweiten Richtung 25 relativ zu dem Kopplungselement 7 bewegt. Das Eingriffselement 34 des Kopplungselements 7 fährt dabei die Kulissenführung 17 in der entgegengesetzten Richtung wie in Figur 1B ab, wodurch das Kopplungselement 7 aus der Ruheposition in die in Figur 1E dargestellte ausgelenkte Position 23 angehoben wird. Das Kopplungselement 7 hat das Maximum 19 der Kulissenführung 17 erreicht. Die Bewegung des Kopplungselements 7 wird auf das daran angekoppelte Testelement 4 übertragen, so dass das Testelement 4 ebenfalls angehoben wird und durch diese Bewegung zur Probenaufnahme z. B. mit einer Blutprobe an einem Körperteil eines Patienten in Kontakt gebracht wird.

Gleichzeitig wird durch den Motor 11 über die Bewegung des Antriebselements 15 in der zweiten Richtung 25 die Feder 9 gespannt (Aufladen des mechanischen Energiespeichers 10). Die Energie des Motors 11 wird daher gleichzeitig zum Aufladen des mechanischen Energiespeichers 10 und zur Bewegung des Kulissenkörpers 16 in der zweiten Richtung 25 relativ zu dem Kopplungselement 7 genutzt. Die Kulisse 12 und das Antriebselement 15 sind dabei so gestaltet, dass sie während des Aufladens des mechanischen Energiespeichers 10 Energie des Motors 11 auf das Kopplungselement 7 zum Ausführen der Probenaufnahmebewegung durch das Testelement 4 übertragen. Dies wird insbesondere durch die Gestaltung der Kopplung der Kulisse 12, des Motors 11 und des mechanischen Energiespeichers 10 an das Antriebselement 15 erreicht.

Figur 1F zeigt das erfindungsgemäße Probengewinnungssystem aus Figuren 1A bis 1E nach dem Ausführen einer Probenaufnahmebewegung.

Nachdem das Kopplungselement 7 das Maximum 19 der Kulissenführung 17 erreicht hat, wird der Kulissenkörper 16 durch den Motor 11 über das Antriebselement 15 in der zweiten Richtung 25 relativ zu dem Kopplungselement 7 weiterbewegt, wobei gleichzeitig die Feder durch das Antriebselement weiter gespannt wird. Das Eingriffselement 34 des Kopplungselements 7 fährt dabei weiterhin die Kulissenführung 17 ab, bis die Feder 9 vollständig gespannt ist und der Motor 11 gestoppt wird. In diesem Zustand befindet sich das Kopplungselement 7 auf der Höhe des ersten Minimums 26 der Kulissenführung 17, so dass es sich wieder in seiner Ruheposition 22 befindet. Das Testelement 4 ist nun wieder zurückgezogen und hat somit den Probenaufnahmevorgang abgeschlossen. Anschließend wird das Analyseband 2 durch die (nicht dargestellte) Transporteinrichtung in Transportrichtung 6 relativ zu dem Kopplungselement 7 weitertransportiert, bis wieder die erste Position des nächsten, auf dem Analyseband 2 angeordneten analytischen Hilfsmittels 1 erreicht wird. In dieser ersten Position, wie sie in Figur 1F dargestellt ist, ist die Lanzette 3 an das Kopplungselement 7 gekoppelt. Die Feder 9 ist vollständig gespannt und bereit zur Abgabe ihrer Energie zum Durchführen einer weiteren Stechbewegung.

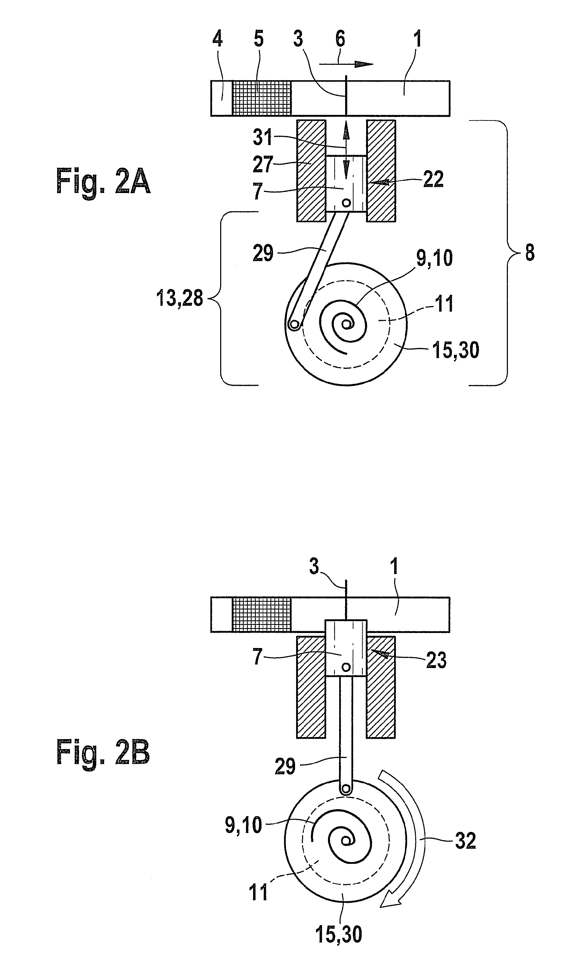

Figur 2A zeigt schematisch eine zweite Ausführungsform eines erfindungsgemäßen Probengewinnungssystems im Ausgangszustand.

Das Probengewinnungssystem enthält ein analytisches Hilfsmittel, wobei es sich um ein einzelnes oder um ein auf einem Analyseband angeordnetes analytisches Hilfsmittel 1 handeln kann. Das analytische Hilfsmittel 1 enthält eine Lanzette 3 und ein Testelement 4. Das Testelement 4 weist ein Testfeld 5 zur Analyse einer flüssigen Probe, insbesondere zur Analyse einer Blutprobe auf. Das analytische Hilfsmittel 1 ist in der Transportrichtung 6 mittels einer (nicht dargestellten) Transportvorrichtung weiterbewegbar. Die Lanzette 3 ist senkrecht zu der Transportrichtung 6 auf dem analytischen Hilfsmittel 1 angeordnet.

Das Probengewinnungssystem enthält ein Kopplungselement 7 in Form eines Stößels, der durch eine Führung 27 in Richtung des analytischen Hilfsmittels geführt wird, wobei das Kopplungselement 7 in Figur 2A an die Lanzette 3 koppelbar ist (erste Position des analytischen Hilfsmittels 1). Das Probengewinnungssystem enthält ferner eine Antriebseinheit 8, um eine Bewegung des Kopplungselements 7 anzutreiben. Die Antriebseinheit 8 umfasst eine Feder 9 als mechanischen Energiespeicher 10, einen Motor 11 und einen Pleuelantrieb 28 als mechanischen Bewegungswandler 13. Die Feder 9 ist gespannt (aufgeladener mechanischer Energiespeicher 10). Der Pleuelantrieb 28 umfasst eine Pleuelstange 29 und einen Antriebsrotor 30, die so mit dem Kopplungselement 7 zusammenwirken, dass eine rotierende Bewegung des Antriebsrotors 30 über die Pleuelstange 29 in eine (durch die Führung 27 geführte) lineare Bewegung 31 des Kopplungselements 7 umgesetzt wird. Der Motor 11 ist an den Antriebsrotor 30 gekoppelt, so dass er diesen bei Bedarf (insbesondere zum Spannen der Feder 9) um einen bestimmten Drehwinkel rotieren kann. Die Feder 9 ist so an den Antriebsrotor 30 gekoppelt, dass sie den Antriebsrotor 30 um einen bestimmten Drehwinkel rotiert, wenn sie von dem gespannten in den entspannten Zustand übergeht. Die Feder 9 gibt dabei die gespeicherte Energie als kinetische Energie an den Antriebsrotor 30 ab. Der Antriebsrotor 30 ist über die Pleuelstange 29 an das Kopplungselement 7 gekoppelt. Daher ist der Antriebsrotor 30 ein Antriebselement 15, das den Motor 11, den mechanischen Energiespeicher 10 (Feder 9) und das Kopplungselement 7 miteinander koppelt. In Figur 2A ist das Kopplungselement 7 in seiner Ruheposition 22 dargestellt.

Figur 2B zeigt das erfindungsgemäße Probengewinnungssystem aus Figur 2A nach dem Auslösen des Stechvorgangs.

Durch das Auslösen entspannt sich die gespannte Feder 9 und der mechanische Energiespeicher 10 gibt seine gespeicherte Energie zumindest teilweise als kinetische Energie über den Antriebsrotor 30 an die Pleuelstange 29 ab. Die Energie der Feder 9 wird dazu auf den Antriebsrotor 30 zum Drehen des Antriebsrotors 30 in einer ersten Drehrichtung 32 übertragen, wodurch die Pleuelstange 29 in Richtung des analytischen Hilfsmittels 1 bewegt wird. Dadurch wird das Kopplungselement 7 (geführt durch die Führung 27) aus der Ruheposition in die in Figur 2B dargestellte ausgelenkte Position 23 angehoben. Die an das Kopplungselement 7 angekoppelte Lanzette 3 wird dadurch ebenfalls angehoben und sticht durch diese Bewegung z. B. in ein Körperteil eines Patienten, um eine Öffnung in der Haut zu erzeugen und eine Blutprobe zu gewinnen. Das Anheben der Lanzette 3 ist in Figur 2B nur schematisch dargestellt, wobei das analytische Hilfsmittel 1 in der Praxis zumindest teilweise mit der Lanzette 3 bewegt wird.

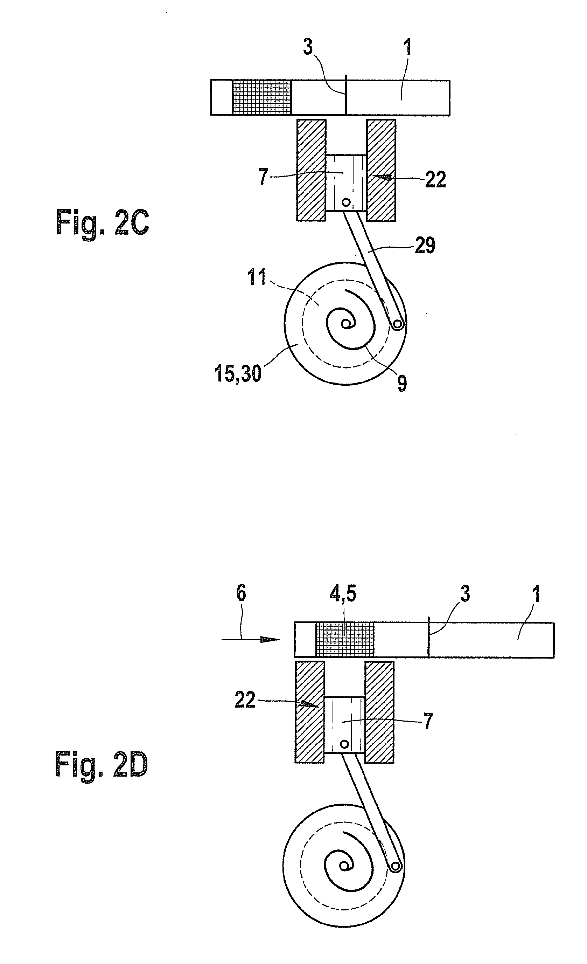

Figur 2C zeigt das erfindungsgemäße Probengewinnungssystem aus Figuren 2A und 2B nach dem Ausführen einer Stechbewegung.

Nachdem das Kopplungselement 7 die maximale Auslenkung erreicht hat (wie in Figur 2B gezeigt), dreht sich der Antriebsrotor 30 durch das weitere Entspannen der Feder 9 noch weiter in der ersten Drehrichtung 32, bis die Feder 9 im wesentlichen vollständig entspannt ist. In diesem Zustand ist die Pleuelstange 29 wieder weiter von dem analytischen Hilfsmittel zurückgezogen, so dass das Kopplungselement ebenfalls wieder zurück in seine Ruheposition 22 bewegt wurde. Dadurch ist die Lanzette 3 wieder zurückgezogen und hat somit ihre Stechbewegung abgeschlossen.

Figur 2D zeigt das erfindungsgemäße Probengewinnungssystem aus Figuren 2A bis 2C, nachdem das analytische Hilfsmittel weitertransportiert wurde.

Eine (nicht dargestellte) Transporteinrichtung transportiert das analytische Hilfsmittel 1 nach der erfolgten Stechbewegung der Lanzette 3 in Transportrichtung 6 relativ zu dem in Ruheposition 22 angeordneten Kopplungselement 7 aus der ersten in die zweite Position, in der das Kopplungselement 7 an das Testelement 4 koppelbar ist.

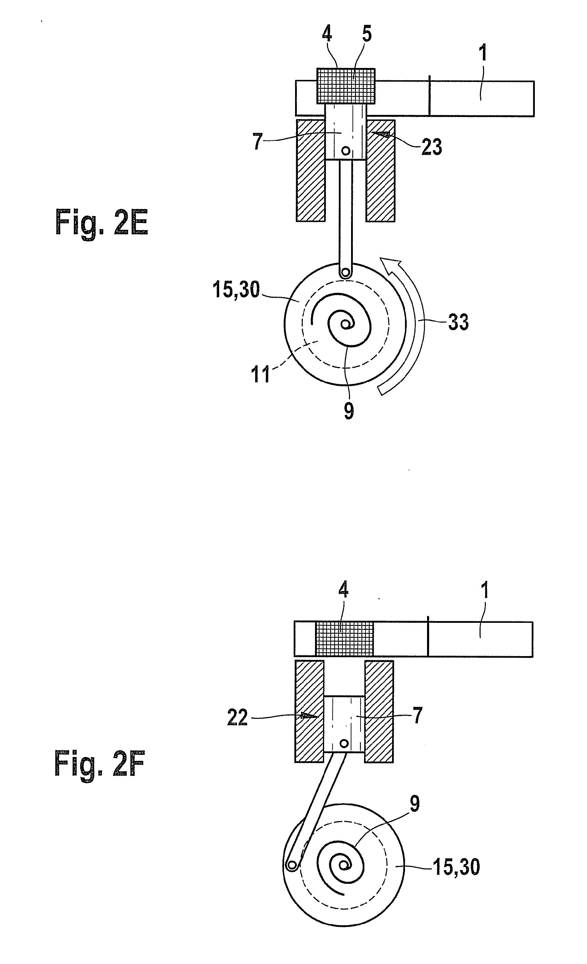

Figur 2E zeigt das erfindungsgemäße Probengewinnungssystem aus Figuren 2A bis 2D beim Ausführen der Probenaufnahmebewegung durch das Testelement.

Das Testelement 4 wird an das Kopplungselement 7 gekoppelt. Der Motor 11 bewegt den Antriebsrotor 30 (Antriebselement 15) in der zweiten Drehrichtung 33, die der ersten Drehrichtung 32 entgegengesetzt ist. Dadurch wird die an den Antriebsrotor 30 gekoppelte Pleuelstange 29 wieder in Richtung des analytischen Hilfsmittels bewegt, wodurch das Kopplungselement 7 (geführt durch die Führung 27) aus der Ruheposition in die in Figur 2E dargestellte ausgelenkte Position 23 angehoben wird. Die Bewegung des Kopplungselements 7 wird auf das daran angekoppelte Testelement 4 übertragen, so dass das Testelement 4 ebenfalls angehoben wird und durch diese Bewegung zur Probenaufnahme z. B. mit einer Blutprobe an einem Körperteil eines Patienten in Kontakt gebracht wird. Das Anheben des Testelements 4 ist in Figur 2E nur schematisch durch Anheben des Testfeldes 5 dargestellt, wobei das analytische Hilfsmittel 1 in der Praxis zumindest teilweise, vorzugsweise vollständig, mit dem Testelement 4 bewegt wird.

Gleichzeitig wird durch den Motor 11 über die Drehung des Antriebsrotors 30 (Antriebselement 15) in der zweiten Drehrichtung 33 die (nicht dargestellte) Feder 9 gespannt (Aufladen des mechanischen Energiespeichers 10). Die Energie des Motors 11 wird daher gleichzeitig zum Aufladen des mechanischen Energiespeichers 10 und zur Bewegung der Pleuelstange 29 genutzt. Der Antriebsrotor 30 und die Pleuelstange 29 werden dabei so gestaltet, dass sie während des Aufladens des mechanischen Energiespeichers 10 Energie des Motors 11 auf das Kopplungselement 7 zum Ausführen der Probenaufnahmebewegung durch das Testelement 4 übertragen. Dies wird insbesondere durch die Gestaltung der Kopplung der Pleuelstange 29, des Motors 11 und des mechanischen Energiespeichers 10 an den Antriebsrotor 30 (das Antriebselement 15) erreicht.

Figur 2F zeigt das erfindungsgemäße Probengewinnungssystem aus Figuren 2A bis 2E nach dem Ausführen einer Probenaufnahmebewegung.

Nachdem das Kopplungselement 7 die maximale Auslenkung (wie in Figur 2E dargestellt) erreicht hat, wird der Antriebsrotor 30 durch den Motor 11 in der zweiten Drehrichtung 33 weiterbewegt. Die Pleuelstange 29 wird dadurch wieder zurückgezogen, bis die Feder 9 vollständig gespannt ist und der Motor 11 gestoppt wird. In diesem Zustand befindet sich das Kopplungselement 7 wieder in seiner Ruheposition 22. Das Testelement 4 ist nun wieder zurückgezogen und hat somit den Probenaufnahmevorgang abgeschlossen. Der Bewegungsablauf aus Figuren 2A bis 2F kann sich nun mit einem neuen analytischen Hilfsmittel 1 wiederholen.

### Bezugszeichenliste

- 1: analytisches Hilfsmittel
- 2: Analyseband
- 3: Lanzette
- 4: Testelement
- 5: Testfeld
- 6: Transportrichtung
- 7: Kopplungselement
- 8: Antriebseinheit
- 9: Feder
- 10: mechanischer Energiespeicher
- 11: Motor
- 12: Kulisse
- 13: mechanischer Bewegungswandler
- 14: Mitnehmer
- 15: Antriebselement
- 16: Kulissenkörper
- 17: Kulissenführung
- 18: Minima
- 19: Maximum
- 20: Bewegungsrichtung des Kulissenkörpers
- 21: erste Richtung
- 22: Ruheposition
- 23: ausgelenkte Position
- 24: zweites Minimum
- 25: zweite Richtung
- 26: erstes Minimum
- 27: Führung
- 28: Pleuelantrieb
- 29: Pleuelstange
- 30: Antriebsrotor
- 31: lineare Bewegung
- 32: erste Drehrichtung
- 33: zweite Drehrichtung
- 34: Eingriffselement

## Patentansprüche

1. Probengewinnungssystem zum Gewinnen einer flüssigen Probe enthaltend mindestens ein analytisches Hilfsmittel (1), wobei das analytische Hilfsmittel (1) eine Lanzette (3) und ein Testelement (4) enthält und das Testelement (4) ein Testfeld (5) zur Analyse der flüssigen Probe aufweist, **dadurch gekennzeichnet, dass** das Probengewinnungssystem
• ein Kopplungselement (7) enthält, das nacheinander in einer ersten Position des analytischen Hilfsmittels (1) an die Lanzette (3) und in einer zweiten Position des analytischen Hilfsmittels (1) an das Testelement (4) koppelbar ist und
• eine Antriebseinheit (8) enthält, um eine Bewegung des Kopplungselements (7) aus einer Ruheposition (22) in eine ausgelenkte Position (23) anzutreiben, so dass die Bewegung des Kopplungselements (7) auf die daran angekoppelte Lanzette (3) zum Ausführen einer Stechbewegung oder auf das daran angekoppelte Testelement (4) zum Ausführen einer Probenaufnahmebewegung übertragen wird.

2. Probengewinnungssystem gemäß Anspruch 1, **gekennzeichnet durch** eine Transporteinrichtung, um das analytische Hilfsmittel (1) relativ zu dem in der Ruheposition (22) angeordneten Kopplungselement (7) aus der ersten Position in die zweite Position zu transportieren.

3. Probengewinnungssystem gemäß einem der Ansprüche 1 oder 2, **dadurch gekennzeichnet, dass** die Antriebseinheit (8) einen mechanischen Energiespeicher (10) enthält, der seine Energie an das Kopplungselement (7) zur Bewegung in die ausgelenkte Position (23) abgeben kann.

4. Probengewinnungssystem gemäß Anspruch 3, **dadurch gekennzeichnet, dass** der mechanische Energiespeicher (10) eine Feder (9) ist.

5. Probengewinnungssystem gemäß einem der Ansprüche 3 oder 4, **dadurch gekennzeichnet, dass** die Antriebseinheit (8) einen Motor (11) zum Aufladen des mechanischen Energiespeichers (10) enthält.

6. Probengewinnungssystem gemäß einem der Ansprüche 3 bis 5, **dadurch gekennzeichnet, dass** die Antriebseinheit (8) so gestaltet ist, dass sie während des Aufladens des mechanischen Energiespeichers (10) Energie auf das Kopplungselement (7) zum Ausführen der Probenaufnahmebewegung durch das Testelement (4) überträgt.

7. Probengewinnungssystem gemäß einem der Ansprüche 3 bis 6, **dadurch gekennzeichnet, dass** die Antriebseinheit (8) einen mechanischen Bewegungswandler (13) umfasst, wobei das Kopplungselement (7) über den mechanischen Bewegungswandler (13) an den mechanischen Energiespeicher (10) gekoppelt ist.

8. Probengewinnungssystem gemäß Anspruch 7, **dadurch gekennzeichnet, dass** der mechanische Bewegungswandler (13) so gestaltet ist, dass er Energie des mechanischen Energiespeichers (10) auf das Kopplungselement (7) zum Ausführen der Stechbewegung mit der Lanzette (3) überträgt.

9. Probengewinnungssystem gemäß einem der Ansprüche 7 oder 8, **dadurch gekennzeichnet, dass** der mechanische Bewegungswandler (13) einen Kulissenkörper (16) mit einer Kulissenführung (17) aufweist, in die ein an dem Kopplungselement (7) angeordnetes Eingriffselement (34) eingreift.

10. Probengewinnungssystem gemäß Anspruch 9, **dadurch gekennzeichnet, dass** der mechanische Energiespeicher (13) und ein Motor (11) über ein Antriebselement (15) an den Kulissenkörper (16) gekoppelt sind, so dass die Energie des mechanischen Energiespeichers (10) auf den Kulissenkörper (16) zur Bewegung des Kulissenkörpers (16) in einer ersten Richtung (21) relativ zu dem Kopplungselement (7) übertragen wird und Energie des Motors (11) während des Aufladens des mechanischen Energiespeichers (10) auf den Kulissenkörper (16) zur Bewegung des Kulissenkörpers (16) in einer zweiten, der ersten entgegengesetzten Richtung (25) relativ zu dem Kopplungselement (7) übertragen werden kann.

11. Probengewinnungssystem gemäß einem der Ansprüche 7 oder 8, **dadurch gekennzeichnet, dass** der mechanische Bewegungswandler (13) als Pleuelantrieb (28) aufgebaut ist, wobei der Pleuelantrieb (28) eine Pleuelstange (29) und einen Antriebsrotor (30) aufweist, die so mit dem Kopplungselement (7) zusammenwirken, dass eine rotierende Bewegung des Antriebsrotors (30) in eine lineare Bewegung (31) des Kopplungselements (7) umgesetzt werden kann.

12. Probengewinnungssystem gemäß Anspruch 11, **dadurch gekennzeichnet, dass** der mechanische Energiespeicher (10) und ein Motor (11) an den Antriebsrotor (30) gekoppelt sind, so dass Energie des mechanischen Energiespeichers (10) auf den Antriebsrotor (30) zum Drehen des Antriebsrotors (30) in einer ersten Drehrichtung (32) übertragen werden kann und Energie des Motors (11) während des Aufladens des mechanischen Energiespeichers (10) auf den Antriebsrotor (30) zum Drehen des Antriebsrotors (30) in eine zweite, der ersten entgegengesetzte Richtung (33) übertragen werden kann.

13. Probengewinnungssystem gemäß einem der Ansprüche 1 bis 12, **gekennzeichnet durch** eine Vielzahl von analytischen Hilfsmitteln (1) auf einem Analyseband (2).

14. Probengewinnungssystem zum Gewinnen einer flüssigen Probe, enthaltend mindestens ein analytisches Hilfsmittel (1), das eine Lanzette (3) und ein Testelement (4) aufweist, und einen mechanischen Energiespeicher (10), der Energie an die Lanzette (3) zum Ausführen einer Stechbewegung abgeben kann, **dadurch gekennzeichnet, dass** das Probengewinnungssystem Übertragungsmittel umfasst, die so an den mechanischen Energiespeicher (10) und das analytische Hilfsmittel (1) gekoppelt sind, dass eine Bewegung der Übertragungsmittel gleichzeitig Energie auf den mechanischen Energiespeicher(10) zum Aufladen des mechanischen Energiespeichers (10) überträgt und eine Bewegung des Testelements (4) antreibt.

15. Probengewinnungssystem gemäß Anspruch 14, **dadurch gekennzeichnet, dass** die Übertragungsmittel durch eine erste Bewegung Energie des mechanischen Energiespeichers (10) auf die Lanzette (3) zum Ausführen der Stechbewegung übertragen und durch eine zweite Bewegung gleichzeitig Energie zum Aufladen des mechanischen Energiespeichers (10) übertragen und die Bewegung des Testelements (4) antreiben.

16. Probengewinnungssystem gemäß einem der Ansprüche 14 oder 15, **dadurch gekennzeichnet, dass** die Übertragungsmittel einen Pleuelantrieb (28) oder einen Kulissenkörper (16) mit einer Kulissenführung (17) umfassen.

17. Verfahren zum Gewinnen einer flüssigen Probe in einem Probengewinnungssystem mittels mindestens eines analytischen Hilfsmittels (1), wobei das analytische Hilfsmittel (1) eine Lanzette (3) und ein Testelement (4) enthält und das Testelement (4) ein Testfeld (5) zur Analyse der flüssigen Probe aufweist, **dadurch gekennzeichnet, dass**
• ein Kopplungselement des Probengewinnungssystems nacheinander in einer ersten Position des analytischen Hilfsmittels (1) an die Lanzette (3) und in einer zweiten Position des analytischen Hilfsmittels (1) an das Testelement (4) gekoppelt wird und
• eine Antriebseinheit eine Bewegung des Kopplungselements aus einer Ruheposition (22) in eine ausgelenkte Position (23) antreibt, so dass die Bewegung des Kopplungselements (7) auf die daran angekoppelte Lanzette (3) zum Ausführen einer Stechbewegung oder auf das daran angekoppelte Testelement (4) zum Ausführen einer Probenaufnahmebewegung übertragen wird.

18. Verfahren zum Gewinnen einer flüssigen Probe in einem Probengewinnungssystem mittels mindestens eines analytischen Hilfsmittels (1), wobei das analytische Hilfsmittel (1) eine Lanzette (3) und ein Testelement (4) enthält, **dadurch gekennzeichnet, dass** eine Bewegung von Übertragungsmitteln des Probengewinnungssystems, die zum Aufladen eines mechanischen Energiespeichers (10) mit Energie zum Antreiben einer Stechbewegung der Lanzette (3) dient, gleichzeitig genutzt wird, um eine Bewegung des Testelements (4) anzutreiben.

19. Verfahren gemäß Anspruch 17 oder 18, **gekennzeichnet durch** die Verfahrensschritte
• Aufnehmen der flüssigen Probe auf das analytische Hilfsmittel (1), das eine Lanzette (3) und ein Testelement (4) mit einem Testfeld (5) zur Analyse der flüssigen Probe umfasst, **durch** Antreiben eines an das Testelement (4) gekoppelten Kopplungselements (7) zum Ausführen einer Probenaufnahmebewegung **durch** das Testelement (4) und
• gleichzeitiges Aufladen eines mechanischen Energiespeichers (10) mit Energie zum Antreiben einer Stechbewegung der an das Kopplungselement (7) gekoppelten Lanzette (3),
wobei das nacheinander an die Lanzette (3) und das Testelement (4) gekoppelte Kopplungselement (7) sowohl bei der Stechbewegung als auch bei der Probenaufnahmebewegung aus einer Ruheposition in eine ausgelenkte Position und zurück in die Ruheposition bewegt wird.
